(19) Europäisches Patentamt
European Patent Office
Office européen des brevets

(11) Veröffentlichungsnummer: **0 072 580**
**B1**

(12) # EUROPÄISCHE PATENTSCHRIFT

(45) Veröffentlichungstag der Patentschrift: **25.02.87**

(21) Anmeldenummer: **82108279.9**

(22) Anmeldetag: **02.05.81**

(51) Int. Cl.⁴: **C 07 D 249/08**, C 07 D 233/60, C 07 D 303/02, C 07 D 303/04, C 07 D 303/08, A 01 N 43/64, A 01 N 43/50

(54) **1-Hydroxyethyl-azol-Derivate, Verfahren zu ihrer Herstellung sowie ihre Verwendung als Pflanzenwachstumsregulatoren und Fungizide.**

(30) Priorität: **16.05.80 DE 3018866**
**19.02.81 DE 3106076**

(43) Veröffentlichungstag der Anmeldung:
**23.02.83 Patentblatt 83/8**

(45) Bekanntmachung des Hinweises auf die Patenterteilung:
**25.02.87 Patentblatt 87/9**

(84) Benannte Vertragsstaaten:
**AT BE CH DE FR GB IT LI NL SE**

(56) Entgegenhaltungen:
**EP-A-0 019 189**
**EP-A-0 052 424**
**EP-A-0 057 357**
**AT-B-346 850**
**CH-A-614 201**
**GB-A-1 156 042**
**US-A-4 123 542**

**TETRAHEDRON LETTERS, Nr. 6, Februar 1976, Seiten 457-460, Pergamon Press, Oxford, G.B. D. VAN ENDE et al.: "Regiospecific synthesis of alpha-beta-unsaturated epoxides (1)"**
**ANGEWANDTE CHEMIE, International Edition, Band 13, Nr. 4, April 1974, Seiten 274-275, Weinheim, DE. W. DUMONT et al.: "Synthesis and reactions of unstabilized alpha-selenonio alkylides"**

(73) Patentinhaber: **BAYER AG, Konzernverwaltung RP Patentabteilung, D-5090 Leverkusen 1 Bayerwerk (DE)**

(72) Erfinder: **Holmwood, Graham, Dr., Katernbergerstrasse 184, D-5600 Wuppertal 1 (DE)**
Erfinder: **Büchel, Karl Heinz, Prof. Dr., Dabringhausener Strasse 42, D-5093 Burscheid (DE)**
Erfinder: **Lürssen, Klaus, Dr., August- Kierspel-Strasse 89, D-5060 Bergisch- Gladbach 2 (DE)**
Erfinder: **Frohberger, Paul- Ernst, Dr., Willi-Baumeister- Strasse 5, D-5090 Leverkusen (DE)**
Erfinder: **Brandes, Wilhelm, Dr., Eichendorffstrasse 3, D-5653 Leichlingen (DE)**

(56) Entgegenhaltungen: (Fortsetzung)
**Die Akte enthält technische Angaben, die nach dem Eingang der Anmeldung eingereicht wurden und die nicht in dieser Patentschrift enthalten sind.**

EP 0 072 580 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue 1-Hydroxyethyl-azol-Derivate, ein Verfahren zu ihrer Herstellung sowie ihre Verwendung als Pflanzenwachstumsregulatoren und Fungizide. Die Erfindung betrifft außerdem neue Oxirane und ein Verfahren zu deren Herstellung.

Es ist bereits bekannt geworden, daß bestimmte 2-Halogen-ethyl-trialkyl-ammonium-halogenide pflanzenwachstumsregulierende Eigenschaften aufweisen (vergleiche US-A-3 156 554). So läßt sich z. B. mit Hilfe von 2-Chlorethyl-trimethyl-ammoniumchlorid eine Beeinflussung des Pflanzenwachstums, insbesondere eine Hemmung des vegetativen Pflanzenwachstums bei wichtigen Kulturpflanzen erzielen. Allerdings ist die Wirksamkeit dieses Stoffes, vor allem bei niedrigen Aufwandmengen, nicht immer ausreichend.

Es ist weiterhin bekannt, daß die 2-Chlorethylphosphon-säure eine pflanzenwachstumsregulierende Wirkung aufweist (vergleiche DE-A-1 667 968). Die mit dieser Substanz erzielten Ergebnisse sind jedoch ebenfalls nicht immer zufriedenstellend.

Ferner ist bereits bekannt geworden, daß Zink-ethylen-1,2-bisdithiocarbamidat ein gutes Mittel zur Bekämpfung von pilzlichen Pflanzenkrankheiten ist [vgl. Phytopathology 33, 1113 (1963)]. Jedoch ist dessen Einsatz nur beschränkt möglich, da es insbesondere bei niedrigen Aufwandmengen und -Konzentrationen nicht immer befriedigend wirksam ist.

Aus der US-A-4 123 542 sind N-Alkyl-imidazole mit fungiziden Eigenschaften bekannt. Es werden jedoch keine Verbindungen erwähnt, die einen über Ethenylen gebundenen substituierten Phenylrest enthalten.

In der EP-A-0 019 189 werden Hydroxyalkyltriazole mit fungizider Wirksamkeit beschrieben. Die allgemeine Formel umfaßt aber ebenfalls keine Stoffe, in denen ein über Ethenylen verknüpfter Phenylrest vorhanden ist.

Die EP-A-0 052 424 und die EP-A 0-057 357 betreffen unter anderem auch solche 1-Hydroxyethyl-azol-Derivate wie sie im vorliegenden Fall beansprucht werden. Diese Druckschriften basieren jedoch auf Anmeldungen, die erst nach dem Prioritätstag der vorliegenden Anmeldung eingereicht wurden.

Schließlich sind aus der US-A-4 123 542, der AT-B-346 850, der CH-A-614 201, der GB-A-1 156 042 sowie aus den Artikeln in Tetrahedron Letters 1976, 457-460 und Angew. Chem. Int. Ed. 13, 274 - 275 (1974) zahlreiche Oxirane bekannt. Es werden aber keine Verbindungen dieses Typs offenbart, die an einem der beiden Kohlenstoffatome des Dreiringes geminal substituiert sind durch einen über Ethenylen verknüpfbaren Phenylrest einerseits und einen tert.-Butyl-, Isopropyl- oder einen gegebenenfalls durch Methyl substituierten Cyclopropyl-Rest andererseits.

Es wurden neue 1-Hydroxyethyl-azol-Derivate der Formel (I)

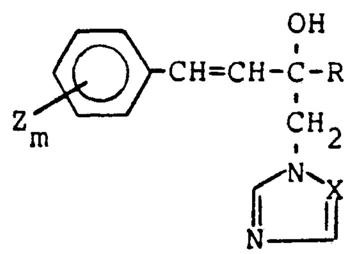

(I)

in welcher

R für Alkyl mit 1 bis 4 Kohlenstoffatomen, gegebenenfalls durch Alkyl mit 1 oder 2 Kohlenstoffatomen substituiertes Cycloalkyl mit 3 bis 7 Kohlenstoffatomen oder für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenyl steht,

X für ein Stickstoffatom oder die CH-Gruppe steht,

Z für Halogen, Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/ oder Alkyl mit 1 bis 4 Kohlenstoffatomen substituiertes Phenylalkyl mit 1 oder 2 Kohlenstoffatomen im Alkylteil oder für gegebenenfalls durch Halogen und/oder Alkyl mit 1 bis 4 Kohlenstoffatomen substituiertes Phenylalkoxy mit 1 oder 2 Kohlenstoffatomen im Alkoxyteil steht und

m für die Zahlen 0, 1, 2 oder 3 steht,

sowie deren Säureadditions-Salze und Metallsalz-Komplexe gefunden.

Die Verbindungen der Formel (I) besitzen ein asymmetrisches Kohlenstoffatom und können deshalb in den

beiden optischen Isomerenformen anfallen.

Außerdem können die Verbindungen der Formel (I) zusätzlich in zwei geometrischen Isomerenformen vorliegen. Die Erfindung betrifft sowohl die Isomerengemische als auch die einzelnen Isomeren.

Weiterhin wurde gefunden, daß man die 1-Hydroxyethyl-azol-Derivate der Formel (I) erhält, wenn man Oxirane der Formel (II)

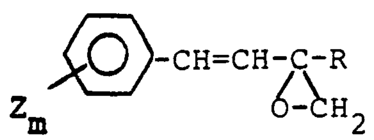

(II)

in welcher
R, Z und m die oben angegebene Bedeutung haben,
mit Azolen der Formel (III)

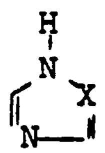

(III)

in welcher
X die oben angegebene Bedeutung hat,
in Gegenwart eines Verdünnungsmittels und gegebenenfalls in Gegenwart einer Base umsetzt, und gegebenenfalls anschließend an die so erhaltenen Verbindungen der Formel (I) eine Säure oder ein Metallsalz addiert.

Außerdem wurde gefunden, daß die neuen 1-Hydroxyethyl-azol-Derivate der Formel (I) starke pflanzenwachstumsregulierende und starke fungizide Eigenschaften besitzen.

Ueberraschenderweise zeigen die erfindungsgemäßen 1-Hydro-xy-azol-Derivate der Formel (I) eine bessere pflanzenwachstumsregulierende Wirkung als das bekannte 2-Chlorethyl-trimethylammoniumchlorid und als die ebenfalls bekannte 2-Chlorethylphosphonsäure, welches anerkannt gut wirksame Stoffe gleicher Wirkungsart sind. Außerdem besitzen die erfindungsgemäßen Verbindungen überraschenderweise eine bessere fungizide Wirkung, als das aus dem Stand der Technik bekannte Zink-ethylen-1,2-bisdithiocarbamidat, welches eine wirkungsmäßig naheliegende Verbindung ist. Im übrigen weisen die erfindungsgemäßen Stoffe überraschenderweise auch bessere pflanzenwachstumsregulierende und fungizide Eigenschaften auf als die konstitutionell ähnlichsten Verbindungen, die aus der DE-A-2 654 890, der DE-A-2 736 122 und der US-A-4 123 542 bekannt sind.

Die erfindungsgemäßen 1-Hydroxyethyl-azol-Derivate sind durch die Formel (I) allgemein definiert. - Besonders bevorzugt sind diejenigen Verbindungen der Formel (I), in denen R für tert.-Butyl, Isopropyl oder Methyl steht, für jeweils gegebenenfalls durch Methyl substituiertes Cyclopropyl, Cyclopentyl oder Cyclohexyl steht sowie für gegebenenfalls einfach oder zweifach, gleich oder verschieden durch Fluor, Chlor, Methyl oder Trifluormethyl substituiertes Phenyl steht; Z für Fluor, Chlor, Brom, Methyl, tert.-Butyl, Cyclohexyl, Methoxy, Methylthio, Trifluormethyl, Trifluormethoxy, Trifluormethylthio sowie für jeweils gegebenenfalls einfach oder zweifach, gleich oder verschieden durch Fluor, Chlor und/oder Methyl substituiertes Phenyl, Phenoxy, Benzyl oder Benzyloxy steht, und X sowie der Index m die in der Erfindungsdefinition angegebene Bedeutung haben.

Im einzelnen seien außer den bei den Herstellungsbeispielen genannten Verbindungen folgende Verbindungen der Formel (I) genannt:

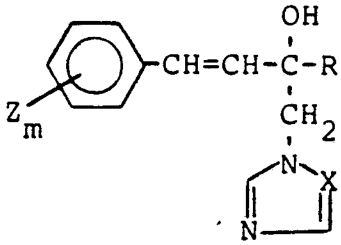

(I)

Tabelle 1

| $Z_m$ | R | X |
|---|---|---|
| 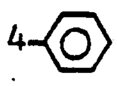 | $-C(CH_3)_3$ | N(CH) |
| 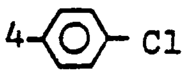 | " | " |
| 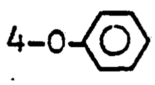 | " | " |
| 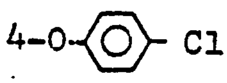 | " | " |
| 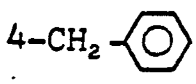 | " | " |
| 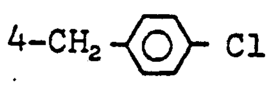 | " | " |
| 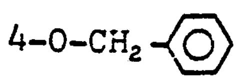 | " | " |
| 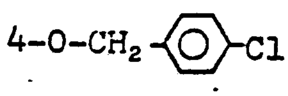 | " | " |
| $3,4-Cl_2$ | " | " |
| $4-CF_3$ | " | " |
| $4-OCF_3$ | " | " |
| $4-SCF_3$ | " | " |
| $4-SCH_3$ | " | " |
| $4-C(CH_3)_3$ | " | " |

<u>Tabelle 1</u> (Fortsetzung)

| $Z_m$ | R | X |
|---|---|---|
| 4-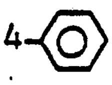 | 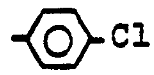 | N(CH) |
| 4-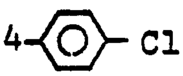 | " | " |
| 4-O-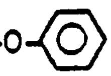 | " | " |
| 4-O-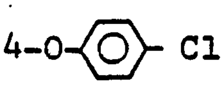 | " | " |
| 4-$CH_2$-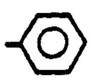 | " | " |
| 4-$CH_2$-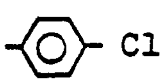 | " | " |
| 4-O-$CH_2$-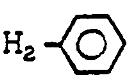 | " | " |
| 4-O-$CH_2$-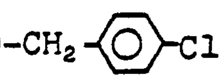 | " | " |
| 3,4-$Cl_2$ | " | " |
| 4-$CF_3$ | " | " |
| 4-$OCF_3$ | " | " |
| 4-$SCF_3$ | " | " |
| 4-$SCH_3$ | " | " |
| 4-$C(CH_3)_3$ | " | " |

<u>Tabelle 1</u> (Fortsetzung)

| $Z_m$ | R | X |
|---|---|---|
| 4-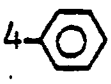 | $-CH(CH_3)_2$ | N(CH) |
| 4-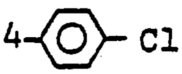 | " | " |
| 4-O-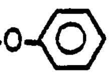 | " | " |
| 4-O-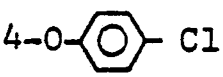 | " | " |
| 4-$CH_2$-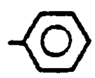 | " | " |
| 4-$CH_2$-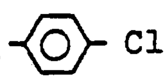 | " | " |
| 4-O-$CH_2$-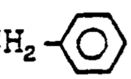 | " | " |
| 4-O-$CH_2$-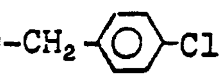 | " | " |
| 3,4-$Cl_2$ | " | " |
| 4-$CF_3$ | " | " |
| 4-$OCF_3$ | " | " |
| 4-$SCF_3$ | " | " |
| 4-$SCH_3$ | " | " |
| 4-$C(CH_3)_3$ | " | " |

<u>Tabelle 1</u> (Fortsetzung)

| $Z_m$ | R | X |
|---|---|---|
| 4-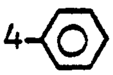 | 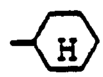 | N(CH) |
| 4-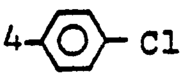 | .. | .. |
| 4-O-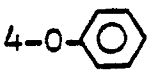 | .. | .. |
| 4-O-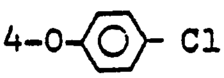 | .. | .. |
| $4-CH_2-$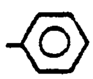 | .. | .. |
| $4-CH_2-$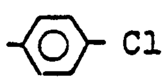 | .. | .. |
| $4-O-CH_2-$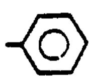 | .. | .. |
| $4-O-CH_2-$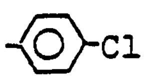 | .. | .. |
| $3,4-Cl_2$ | .. | .. |
| $4-CF_3$ | .. | .. |
| $4-OCF_3$ | .. | .. |
| $4-SCF_3$ | .. | .. |
| $4-SCH_3$ | .. | .. |
| $4-C(CH_3)_3$ | .. | .. |

Tabelle 1 (Fortsetzung)

| $Z_m$ | R | X |
|---|---|---|
| 4- 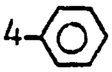 | 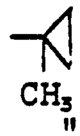 | N (CH) |
| 4- 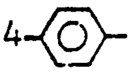 Cl | " | " |
| 4-O- 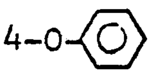 | " | " |
| 4-O- 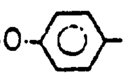 Cl | " | " |
| 4-$CH_2$- 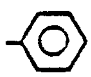 | " | " |
| 4-$CH_2$- 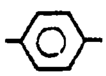 Cl | " | " |
| 4-O-$CH_2$- 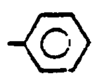 | " | " |
| 4-O-$CH_2$- 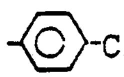 -Cl | " | " |
| 3,4-$Cl_2$ | " | " |
| 4-$CF_3$ | " | " |
| 4-$OCF_3$ | " | " |
| 4-$SCF_3$ | " | " |
| 4-$SCH_3$ | " | " |
| 4-$C(CH_3)_3$ | " | " |

Tabelle 1 (Fortsetzung)

| $Z_m$ | R | X |
|---|---|---|
| 4-Cl | $-CH(CH_3)_2$ | N(CH) |
| 4-F | " | " |
| $4-CH_3$ | " | " |
| 4-Cl | 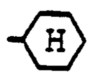 | N (CH) |
| 4-F | " | " |
| $4-CH_3$ | " | " |

Tabelle 1 (Fortsetzung)

| $Z_m$ | R | X |
|---|---|---|
| 4-Cl | 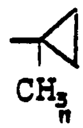 | N(CH) |
| 4-F | " | " |
| $4-CH_3$ | " | " |
| $2,4-Cl_2$ | $-C(CH_3)_3$ | N(CH) |
| $4-CH_3$ | " | " |
| 4-Cl, $2-CH_3$ | " | " |
| 4-F | $-C(CH_3)_3$ | N |

Verwendet man beispielsweise 2-(4-Chlorphenyl-ethenyl)-2-tert.-butyl-oxiran und Imidazol als Ausgangsstofe, so kann der Ablauf des erfindungsgemäßen Verfahrens durch das folgende Formelschema wiedergegeben werden:

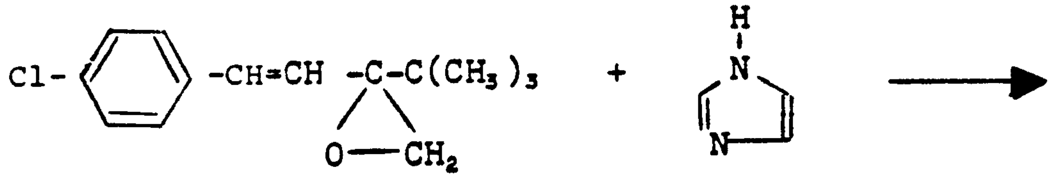

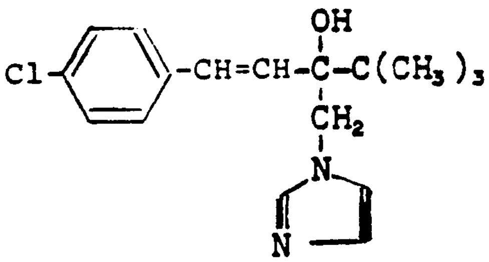

Die bei der Durchführung des erfindungegemäßen Verfahrens als Ausgangsstoffe zu verwendenden Oxirane sind durch die Formel (II) allgemein definiert. In dieser Formel haben R, Z und der Index m vorzugeweise die Bedeutungen, die bereits im Zusammenhang mit der Beschreibung der erfindungsgemäßen Stoffe der Formel (I) vorzugsweise für diese Substituenten bzw. für den Index m genannt wurden.

Die Oxirane der Formel (II) sind teilweise bekannt [vgl. US-A-4 123 542, AT-B-346 850, CH-A-614 201, GB-A-1 156 042, Tetrahedron Letters 1976, 457 - 460 und Angew. Chem. Int. Ed. 13, 274 - 275 (1974)].

Oxirane der Formel (IIa)

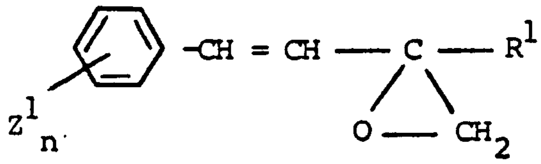

(IIa)

in welcher

$R^1$ für tert.-Butyl, Isopropyl oder für gegebenenfalls durch Methyl substituiertes Cyclopropyl steht,

$Z^1$ für Fluor, Chlor, Brom, Methyl, tert.-Butyl, Cyclohexyl, Methoxy, Methylthio, Trifluormethyl, Trifluormethoxy, Trifluormethylthio, Phenyl, Phenoxy, Benzyl oder Benzyloxy steht und

n für die Zahlen 0, 1 oder 2 steht,

sind neu.

Die Oxirane der Formel (IIa) lassen sich herstellen, indem man Ketone der Formel (IV)

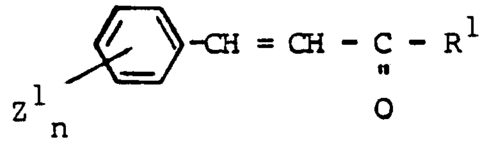 (IV)

in welcher
$R^1$, $Z^1$ und n die oben angegebene Bedeutung haben,
entweder
a) mit Dimethyloxosulfonium-methylid der Formel (V)

$$\overset{\delta+}{(CH_3)_2SO}\overset{\delta-}{CH_2} \qquad (V)$$

in Gegenwart eines Verdünnungsmittels umsetzt,
oder
b) mit Trimethylsulfoniummethylsulfat der Formel (VI)

$$\left[(CH_3)_3S\right]^{(+)} \; CH_3SO_4^{(-)} \qquad (VI)$$

in Gegenwart eines inerten organischen Lösungsmittels sowie in Gegenwart einer Base umsetzt.

Die übrigen Oxirane der Formel (II) lassen sich in analoger Weise herstellen.

Die bei der Herstellung der Oxirane der Formel (IIa) als Ausgangsstoffe benötigten Ketone der Formel (IV) sind bekannt [vgl. DE-C-22 01 063, DE-A-27 05 678, DE-A-27 37 489, Tetrahedron 31, 3 (1975) und Chemical Abstracts 84, 73 906 n] oder lassen sich nach im Prinzip bekannten Verfahren herstellen.

Das bei der Verfahrensvariante a) benötigte Dimethyloxosulfonium-methylid der Formel (V) ist ebenfalls bekannt [vgl. J. Amer. Chem. Soc. 87, 1363-1364 (1965)]. Es wird bei der obigen Umsetzung in frisch zubereitetem Zustand verarbeitet, indem man es in situ durch Umsetzung von Trimethyloxosulfoniumjodid mit Natriumhydrid bzw. Natriumamid in Gegenwart eines Verdünnungsmittels erzeugt.

Das bei der Verfahrensvariante b) benötigte Trimethylsulfonium-methylsulfat der Formel (VI) ist ebenfalls bekannt [vgl. Heterocycles 8, 397 (1977)]. Es wird bei der obigen Umsetzung ebenfalls in frisch hergestelltem Zustand eingesetzt, indem man es durch Reaktion von Dimethylsulfid mit Dimethylsulfat in situ erzeugt.

Bei der Variante a) des Verfahrens zur Herstellung der Oxirane der Formel (II) kommt als Verdünnungsmittel vorzugsweise Dimethylsulfoxid in Frage.

Die Reaktionstemperaturen können bei der oben beschriebenen Verfahrensvariante a) innerhalb eines größeren Bereiches variiert werden. Im allgemeinen arbeitet man bei Temperaturen zwischen 20 und 80°C.

Die Durchführung des Verfahrens zur Herstellung der Oxirane der Formel (IIa) nach der Variante a) und die Aufarbeitung des bei dieser Synthese anfallenden Reaktionsgemisches erfolgen nach üblichen Methoden [vgl.

J. Amer. Chem. Soc. 87, 1363-1364 (1965)]

Bei der Variante b) zur Herstellung der Oxirane der Formel (II) kommt als inertes organisches Lösungsmittel vorzugsweise Acetonitril in Betracht.

Als Basen können bei der Varfahrensvariante b) starke anorganische oder organische Basen verwendet werden. Vorzugsweise in Frage kommt Natriummethylat.

Die Reaktionstemperaturen können bei der oben beschriebenen Verfahrensvariante b) innerhalb eines bestimmten Bereiches variiert werden. Im allgemeinen arbeitet man bei Temperaturen zwischen 0 und 60°C, vorzugsweise bei Raumtemperatur.

Die Durchführung des Verfahrens zur Herstellung der Oxirane der Formel (II) nach der Variante b) und die Aufarbeitung des bei dieser Synthese anfallenden Reaktionsproduktes erfolgen nach üblichen Methoden [vgl. Heterocycles 8, 397 (1977)].

Die Oxirane der Formel (II) können bei dem erfindungsgemäßen Verfahren gegebenenfalls ohne Isolierung direkt weiter umgesetzt werden.

Die außerdem für das erfindungsgemäße Verfahren als Ausgangsstoffe zu verwendenden Azole sind durch die Formel (III) allgemein definiert.

Die Azole der Formel (III) sind allgemein bekannte Verbindungen der organischen Chemie.

Als Verdünnungsmittel kommen für die erfindungsgemäße Umsetzung alle inerten organischen Lösungsmittel infrage. Hierzu gehören vorzugsweise Alkohole, wie z.B. Ethanol und Methoxysthanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für die erfindungsgemäße Umsetzung alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kaliummethylat und -ethylat; Alkalihydride, wie z.B. Natriumhydrid; Sowie niedere tertiäre alkylamine, Cycloalkylamine und Aralkylamine, wie insbesondere Triethylamin.

Die Reaktionatemperaturen können bei der Durchführung des erfindungsgemäßen Verfahrens in einem größeren Bereich variiert werden. Im allgemeinen arbeitet man bei Temperaturen zwischen 0 und 200°C, vorzugsweise zwischen 60 und 150°C.

Die erfindungsgemäße Umsetzung kann gegebenenfalls unter erhöhtem Druck durchgeführt werden. Im allgemeinen arbeitet man zwischen 1 und 50 bar, vorzugsweise zwischen 1 und 25 bar.

Bei der Durchführung des erfindungsgemäßen Verfahrens setzt man auf 1 Mol Oxiran der Formel (II) vorzugsweise 1 bis 2 Mol Azol und gegebenenfalls 1 bis 2 Mol Base ein. Die Isolierung der Endprodukte erfolgt in allgemein üblicher Weise.

Die nach dem erfindungsgemäßen Verfahren erhältlichen Verbindungen der Formel (I) können in Säureadditions-Salze bzw. Metallsalz-Komplexe überführt werden.

Zur Herstellung von physiologisch verträglichen Säureadditionssalzen der Verbindungen der Formel (I) kommen vorzugsweise folgende Säuren in Frage: Die Halogenwasserstoffsäuren, wie z.B. Chlorwasserstoffsäure und die Bromwasserstoffsäure, insbesondere die Chlorwasserstoffsäure, ferner Phosphorsäure, Salpetersäure, Schwefelsäure, monound bifunktionelle Carbonsäuren und Hydroxycarbonsäuren, wie z.B. Essigsäure, Maleinsäure, Bernsteinsäure, Fumarsäure, Weinsäure, Citronensäure, Salicylsäure, Sorbinsäure, Milchsäure sowie Sulfonsäuren, wie z. B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z.B. durch Lösung einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Hauptgruppe und der I. und II. sowie IV. bis VIII. Nebengruppe des Periodensystems in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Scnwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, Isolieren und gegebenenfalls durch Umkristallisieren reinigen.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ('Lagerns') der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngenkengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wüchshemmung sehr erwünscht sein kann. Andererseits ist es aber auch möglich, das Wachstum der Seitentriebe zu hennen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Vorussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Heimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder

hohen Salzgehalt des Bodens induziert werden. Hierdurch wird die Kultivierung von Pflanzen in Gebieten möglich, die hierzu normalerweise ungeeignet sind.

Die erfindungsgemäßen Wirkstoffe weisen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromacetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäß verwendbaren Wirkstoffe mit besonders gutem Erfolg zur Bekämpfung solcher Pilze eingesetzt werden, die echte Mehltauerkrankungen hervorrufen; so zur Bekämpfung von Erysiphe-Arten, wie z.B. gegen den Erreger des Gersten- bzw. des Getreidemehltaus (Erysiphe graminis).

Besonders hervorzuheben ist, daß die erfindungsgemäßen Wirkstoffe nicht nur eine protektive Wirkung entfalten, sondern auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Vervedung von oberflächenaktiven Mitteln, also Emulgienmitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Kylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan, oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetiache Granulate aus anorganischen und organischen Mehlen sowie Granulate aus organischem Material wie Sägemehl, Kokoanußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonste, Alkylsulfate, Arylsulfonste sowie Eiweißhydrolysate. Als Dispergiermittel kommen in Frage: z.B. Lignin-Sulfitsblaugen und Methylcellulose.

Es können in den Formulierungen Haftmittel wie Carboxymethylcellulose, natürliche und synthetische pulverige, körnige oder latexförmige Polymere verwendet verden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgutder Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg bevorzugt 0,05 bis 10 kg an Wirkstoff.

Beim Einsatz der erfindungsgemößen Stoffe als Pflanzenwachstumsregulatoren gilt, daß die Anwendung in einem bevorzugten Zeitraum vorgenommen wird,dessen genaue Abgrenzung sich nach den klimatischen und vegetativen Gegebenheiten richtet.

Auch beim Einsatz der erfindungsgemäßen Stoffe als Fungizide Kann die aufwandmenge je nach Art der Applikation in einem größeren Bereich varriiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-% vorzugsweise von 0,0001 bis 0,02 %, am Wirkungsort erforderlich.

**Herstellungsbeispiele**

**Beispiel 1**

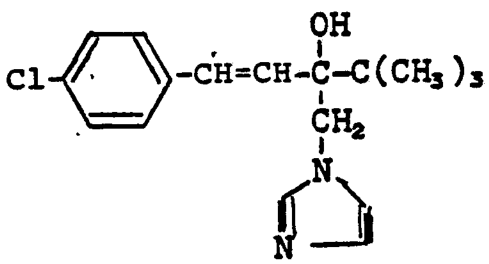

(I-1)

Eine Lösung von 17,75 g (0,075 Mol) 2-(4-Chlorphenyl-ethenyl)-2-tert.-butyl-oxiran und 6,8 g (0,1 Mol) Imidazol in 30 ml Ethanol wird 20 Stunden bei 150° C im Bombenrohr erhitzt. Danach wird das Reaktionsgemisch eingeengt und der kristalline Rückstand mit Ether verrührt. Der Feststoff wird anschließend abgesaugt und aus Acetonitril umkristallisiert. Man erhält 1-(4-Chlorphenyl)-4,4-dimethyl-3-(imidazol-1-yl-methyl)-1-penten-3-ol vom Schmelzpunkt 158,5 - 162° C.

Herstellung des Ausgangsproduktes:

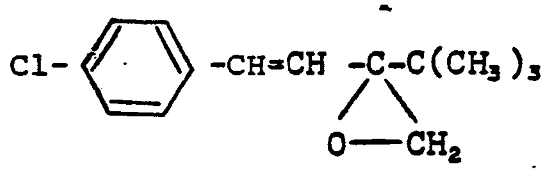

(II-1)

Eine Lösung von 189 ml (0,2 Mol) Dimethylsulfat in 1200 ml absolutem Actonitril wird bei Raumtemperatur mit einer Lösung von 162 ml (2,2 Mol) Dimethylsulfid in 400 ml absolutem Acetonitril versetzt. Man läßt die Reaktionsmischung über Nacht bei Raumtemperatur rühren. Danach werden 118,8 g (2,2 Mol) Natriummethylat zugegeben. Man läßt 30 Minuten rühren und versetzt dann innerhalb von 30 Minuten tropfenweise mit einer Lösung von 267 g (1,2 Mol) 1-(4-Chlorphenyl)-4,4-dimethyl-but-1-en-3-on in 600 ml absolutem Acetonitril. Man läßt das Reaktionsgemisch über Nacht nachrühren. Anschließend wird eingeengt, der Rückstand zwischen Wasser und Essigester verteilt, die organische Phase abgetrennt, zweimal mit Wasser und einmal mit gesättigter Natriumchloridlösung gewaschen, über Natriumsulfat getrocknet, eingeengt und der Rückstand im Vakuum destilliert. Man erhalt 2-(4-Chlorphenyl-ethenyl)-2-tert.-butyl-oxiran in Form einer Festsubstanz vom Schmelzpunkt 61 - 62,5° C.

In analoger Weise wurden die nachfolgenden Verbindungen der Formel (I) erhalten:.

**Tabelle 2**:

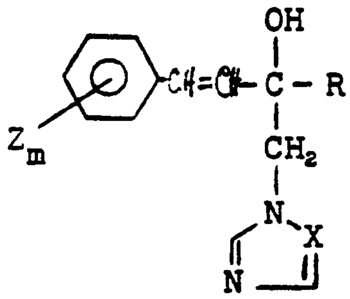

(I)

| Bsp. Nr. | $Z_m$ | R | X | Schmelzpunkt (°C) |
|---|---|---|---|---|
| I-2 | $2-CH_3$ | $-C(CH_3)_3$ | N | Oel |
| I-3 | 4-F | $-C(CH_3)_3$ | CH | 144-146 |
| I-4 | $2-CH_3$ | $-C(CH_3)_3$ | CH | 127-132 |
| I-5 | $4-CH_3$ | $-C(CH_3)_3$ | N | 117-119 |
| I-6 | $4-CH_3$ | $-C(CH_3($3 | CH | 144-146 |
| I-7 | $2,6-Cl_2$ | $-C(CH_3)_3$ | CH | 110-116 |

Entsprechend Beispiel 1 werden die nachfolgenden Zwischenprodukte der allgemeinen Formel (II) erhalten:

**Tabelle 3:**

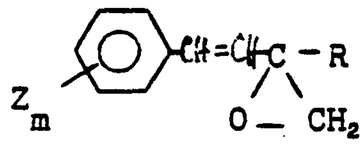

(II)

| Bsp. Nr. | $Z_m$ | R | Siedepunkt(°C)/ mm Hg-Säule |
|---|---|---|---|
| II- 2 | 2,4-$Cl_2$ | $-C(CH_3)_3$ | nicht isoliert |
| II- 3 | 4-$CH_3$ | " | nicht isoliert |
| II- 4 | 4-F | " | 75/0,005 |
| II- 5 | 2-$CH_3$ | " | 71-74/0,01 |
| II- 6 | 2,6-$Cl_2$ | " | nicht isoliert |

Die gute Wirksamkeit der erfindungsgemäßen Stoffe geht aus den nachfolgenden Beispielen hervor.

**Beispiel A**

**Wuchshemmung bei Zuckerrüben**

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird der Zuwachs der Pflanzen gemessen und die Wuchshemmung in Prozent des Zuwachses der Kontrollpflanzen berechnet. Es bedeuten 0 % Wuchshemmung ein Wachstum entsprechend dem der Kontrollpflanzen. 100 % Wuchshemmung bedeutet Stillstand des Wachstums.

Die erfindungsgemäßen Wirkstoffe zeigen in diesem Test eine sehr gute Wuchshemmung.

**Beispiel B**

**Wuchshemmung bei Sojabohnen**

Lösungsmittel: 10 Gewichtsteile Methanol
Emulgator : 2 Gewichtsteile Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Junge Sojabohnenpflanzen werden in dem Stadium, in dem die ersten Folgeblätter entfaltet sind, mit den Wirkstoffzubereitungen tropfnaß besprüht. Nach 2 Wochen wird der Zuwachs gemessen und die Wuchshemmung in % des Zuwachses der Kontrollpflanzen berechnet. Es bedeuten 100 % den Stillstand des Wachstums und 0 % ein Wachstum entsprechend dem der unbehandelten Kontrollpflanzen.

Die erfindungsgemäßen Wirkstoffe zeigen in diesem Test eine sehr gute Wuchshemmung.

**Beispiel C**

**Wuchshemmung bei Baumwolle**

Lösungsmittel: 30 Gewichtsteile Dimethylformamid
Emulgator : 1 Gewichtsteile Polyoxyethylen-Sorbitan-Monolaurat

Zur Herstellung einer zweckmäßigen Wirkstoffzubereitung vermischt man 1 Gewichtsteil Wirkstoff mit der angegebenen Menge Lösungsmittel und Emulgator und füllt mit Wasser auf die gewünschte Konzentration auf.

Baumwollpflanzen werden im Gewächshaus bie zur vollen Entfaltung des 5.Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Die erfindungsgemäßen Wirkstoffe zeigen in diesem Test eine sehr gute Wuchshemmung.

**Beispiel D**

**Stimulierung der $CO_2$-Assimilation bei Sojabohnen**

Sojabohnen werden, wie im Beispiel (B) beschrieben, mit den Wirkstoffzubereitungen behandelt. 7 Tage nach der Behandlung wird an Blattscheiben dieser Pflanzen und entsprechender Kontrollpflanzen die $CO_2$-Assimilation mit Hilfe eines Infrarotanalysators gemessen. Die erfindungsgemäßen Wirkstoffe zeigen in den Konzentrationen 250, 500 und 1000 ppm eine gegenüber den Kontrollen deutlich erhöhte $CO_2$-Assimilation. Dieser Effekt läßt Ertragssteigerungen durch den Wirkstoff erwarten.

**Beispiel E**

Erysiphe-Test (Gerste) / protektiv /
Lösungsmittel: 100 Gewichtsteile Dimethylformamid
Emulgator: 0,25 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

Die erfindungsgemäßen Stoffe zeigen in diesem Test eine sehr gute Wirksamkeit.

**Beispiel F**

Gerstenmehltau-Test (Erysiphe graminis var.hordei)/ systemisch (pilzliche Getreidesproßkrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 x 12 Korn in Blumentöpfe 2 cm tief in ein Gemisch aus einem Volumenteil Pruhstorfer Einheitserde und einem Volumenteil Quarzsand ein. Die Keimung und der auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var.hordei bestäubt und bei 21-22°C und 30-90% rel.Luftfeuchte und 16-stündiger Belichtung weiter kultiviert. Innerhalb von 6 Tagen bilden sich an den Blättern die typischen Mehltaupusteln aus.

Der Befallsgarad wird in Prozfent des Befalls der unbehandelten kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Die erfindungsgemäßen Stoffe zeigen in diesem Test eine sehr gute Wirksamkeit.

**Patentansprüche**

1) 1-Hydroxyethyl-azol-Derivate der Formel (I)

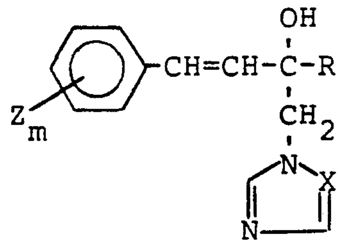

(I)

in welcher
R für Alkyl mit 1 bis 4 Kohlenstoffatomen, gegebenenfalls durch Alkyl mit 1 oder 2 Kohlenstoffatomen substituiertes Cycloalkyl mit 3 bis 7 Kohlenstoffatomen oder für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenyl steht,
X für ein Stickstoffatom oder die CH-Gruppe steht,
Z für Halogen, Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/ oder Alkyl mit 1 bis 4 Kohlenstoffatomen substituiertes Phenylalkyl mit 1 oder 2 Kohlenstoffatomen im Alkylteil oder für gegebenenfalls durch Halogen und/oder Alkyl mit 1 bis 4 Kohlenstoffatomen substituiertes Phenylalkoxy mit 1 oder 2 Kohlenstoffatomen im Alkoxyteil steht und
m für die Zahlen 0, 1, 2 oder 3 steht,
sowie deren Säureadditions-Salze und Metallsalz-Komplexe.

2)Verfahren zur Herstellung von 1-Hydroxyethyl-azol-Derivaten der Formel (I)

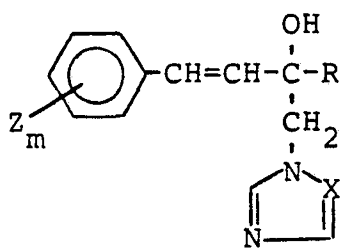

(I)

in welcher
R für Alkyl mit 1 bis 4 Kohlenstoffatomen, gegebenenfalls durch Alkyl mit 1 oder 2 Kohlenstoffatomen substituiertes Cycloalkyl mit 3 bis 7 Kohlenstoffatomen oder für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenyl steht,
X für ein Stickstoffatom oder die CH-Gruppe steht,
Z für Halogen, Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit

1 bis 4 Kohlenstoffatomen substituiertes Phenylalkyl mit 1 oder 2 Kohlenstoffatomen im Alkylteil oder für gegebenenfalls durch Halogen und/oder Alkyl mit 1 bis 4 Kohlenstoffatomen substituiertes Phenylalkoxy mit 1 oder 2 Kohlenstoffatomen im Alkoxyteil steht und

m für die Zahlen 0, 1, 2 oder 3 steht,

sowie deren Säureadditions-Salzen und Metallsalz-Komplexen, dadurch gekennzeichnet, daß man Oxirane der Formel (II)

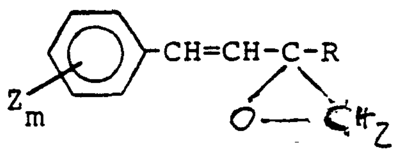

(II)

in welcher

R, Z und m die oben angegebene Bedeutung haben,

mit Azolen der Formel (III)

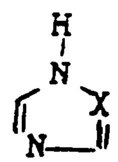

(III)

in welcher

X die oben angegebene Bedeutung hat,

in Gegenwart eines Verdünnungsmittels und gegebenenfalls in Gegenwart einer Base umsetzt, und gegebenenfalls anschließend an die so erhaltenen Verbindungen der Formel (I) eine Säure oder ein Metallsalz addiert.

3) Pflanzenwachstumsregulierende und fungizide Mittel, gekennzeichnet durch einen Gehalt an mindestens einem 1-Hydroxyethyl-azol-Derivat der Formel (I) bzw. einem Säureadditions-Salz oder Metallsalz-Komplex eines 1-Hydroxyethyl-azol-Derivates der Formel (I) neben Streckmitteln und/oder oberflächenaktiven Stoffen.

4) Verwendung von 1-Hydroxyethyl-azol-Derivaten der Formel (I) bzw. von Säureadditions-Salzen oder Metallsalz-Komplexen von 1-Hydroxyethyl-azol-Derivaten der Formel (I) zur Regulierung des Pflanzenwachstums bzw. zur Bekämpfung von Pilzen.

5) Verfahren zur Herstellung von pflanzenwachstumsregulierenden und fungiziden Mitteln, dadurch gekennzeichnet, daß man 1-Hydroxyethyl-azol-Derivate der Formel (I) bzw. Säureadditions-Salze oder Metallsalz-Komplexe von 1-Hydroxyethyl-azol-Derivaten der Formel (I) mit Streckmitteln und/oder oberflächenaktiven Stoffen vermischt.

6) Oxirane der Formel (IIa)

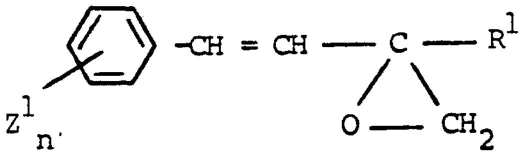 (IIa)

in welcher
$R^1$ für tert.-Butyl, Isopropyl oder für gegebenenfalls durch Methyl substituiertes Cyclopropyl steht,
$Z^1$ für Fluor, Chlor, Brom, Methyl, tert.-Butyl, Cyclohexyl, Methoxy, Methylthio, Trifluormethyl, Trifluormethoxy, Trifluormethylthio, Phenyl, Phenoxy, Benzyl oder Benzyloxy steht und
n für die Zahlen 0, 1 oder 2 steht.
7) Verfahren zur Herstellung von Oxiranen der Formel (IIa)

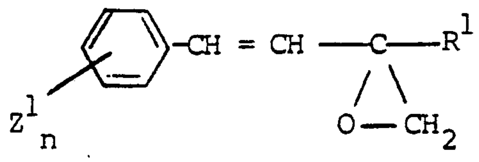 (IIa)

in welcher
$R^1$ für tert.-Butyl, Isopropyl oder für gegeberenfalls durch Methyl substituiertes Cyclopropyl steht,
$Z^1$ für Fluor, Chlor, Brom, Methyl, tert.-Butyl, Cyclohexyl, Methoxy, Methylthio, Trifluormethyl, Trifluormethoxy, Trifluormethylthio, Phenyl, Phenoxy, Benzyl oder Benzyloxy steht und
n für die Zahlen 0, 1 oder 2 steht,
dadurch gekennzeichnet, daß man Ketone der Formel (IV)

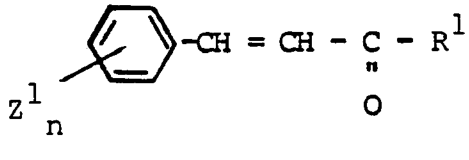 (IV)

in welcher
$R^1$, $Z^1$ und n die oben angegebene Bedeutung haben,
entweder
a) mit Dimethyloxosulfonium-methylid der Formel (V)

$$\overset{\delta+\ \delta-}{(CH_3)_2SOCH_2} \qquad (V)$$

in Gegenwart eines Verdünungsmittels umsetzt,
oder
b) mit Trimethylsulfoniud-methylsulfat der Formel (VI)

$$\left[(CH_3)_3S^{(+)}\right] CH_3SO_4^{(-)} \qquad (VI)$$

in Gegenwart eines inerten organischen Lösungsmittels sowie im Gegenwart einer Base umsetzt.

**Claims**

1. 1-Hydroxyethyl-azole derivatives of the formula (I)

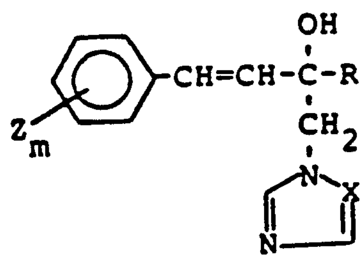

(I)

in which
R represents alkyl with 1 to 4 carbon atoms, cycloalkyl which has 3 to 7 carbon atoms and is optionally substituted by alkyl with 1 or 2 carbon atoms, or phenyl which is optionally substituted by halogen, alkyl with 1 to 4 carbon atoms and/or halogenoalkyl with 1 or 2 carbon atoms and 1 to 5 halogen atoms,
X represents a nitrogen atom or the CH group,
Z represents halogen, alkyl with 1 to 4 carbon atoms, cycloalkyl with 5 to 7 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms, or phenylalkoxy which has 1 or 2 carbon atoms in the alkoxy part and is optionally susbtituted by halogen and/or alkyl with 1 to 4 carbon atoms and
m represents the numbers 0, 1, 2 or 3,
and their acid addition salts and metal salt complexes.

2. Process for the preparation of 1-hydroxyethyl-azole derivatives of the formula (I)

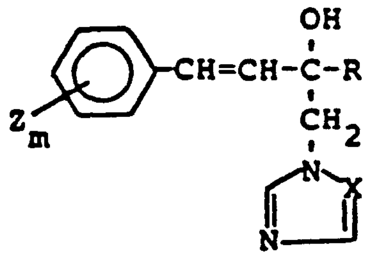

(I)

in which

R represents alkyl with 1 to 4 carbon atoms, cycloalkyl which has 3 to 7 carbon atoms and is optionally substituted by alkyl with 1 or 2 carbon atoms, or phenyl which is optionally substituted by halogen, alkyl with 1 to 4 carbon atoms and/or halogenoalkyl with 1 or 2 carbon atoms and 1 to 5 halogen atoms,

X represents a nitrogen atom or the CH group,

Z represents halogen, alkyl with 1 to 4 carbon atoms, cycloalkyl with 5 to 7 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms or phenylalkoxy which has 1 or 2 carbon atoms in the alkoxy part and is optionally substituted by halogen and/or alkyl with 1 to 4 carbon atoms and

m represents the numbers 0, 1, 2 or 3,

and their acid addition salts and metal salt complexes,

characterised in that oxiranes of the formula (II)

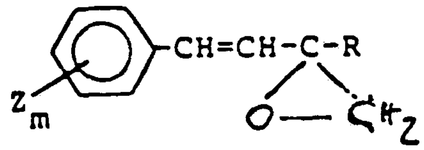

(II)

in which

R, Z and m have the abovementioned meaning, are reacted with azoles of the formula (III)

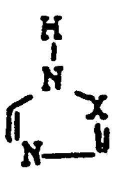

(III)

in which

X has the abovementioned meaning, in the presence of a diluent and, if appropriate, in the presence of a

base, and, if desired, an acid or a metal salt is then added on to the compounds of the formula (I) thus obtained.

3. Plant-growth-regulating and fungicidal agents, characterised in that they contain at least one 1-hydroxyethylazole derivative of the formula (I) or an acid addition salt or metal salt complex of a 1-hydroxyethyl-azole derivative of the formula (I) in addition to extenders and/or surface-active substances.

4. Use of 1-hydroxyethyl-azole derivatives of the formula (I) or acid addition salts or metal salt complexes of 1-hydroxyethyl-azole derivatives of the formula (I) for regulating plant growth or for combating fungi.

5. Process for the preparation of plant-growth-regulating and fungicidal agents, characterised in that 1-hydroxyethylazole derivatives of the formula (I) or acid addition salts or metal salt complexes of 1-hydroxyethyl-azole derivatives of the formula (I) are mixed with extenders and/or surface-active substances.

6. Oxiranes of the formula (IIa)

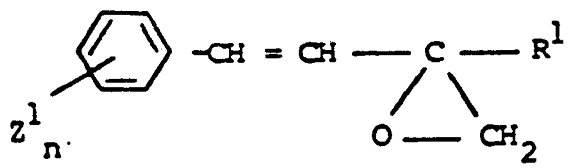

(IIa)

in which

$R^1$ represents tert.-butyl, isopropyl or cyclopropyl which is optionally substituted by methyl,

$Z^1$ represents fluorine, chlorine, bromine, methyl, tert.-butyl, cyclohexyl, methoxy, methylthio, trifluoromethyl, trifluoromethoxy, trifluoromethylthio, phenyl, phenoxy, benzyl or benzyloxy and

n represents the numbers 0, 1 or 2.

7. Process for the preparation of oxiranes of the formula (IIa)

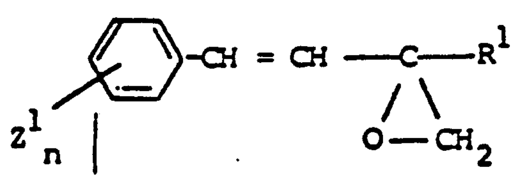

(IIa)

in which

$R^1$ represents tert.-butyl, isopropyl or cyclopropyl which is optionally substituted by methyl,

$Z^1$ represents fluorine, chlorine, bromine, methyl, tert.-butyl, cyclohexyl, methoxy, methylthio, trifluoromethyl, trifluoromethoxy, trifluoromethylthio, phenyl, phenoxy, benzyl or benzyloxy and

n represents the numbers 0, 1 or 2,

characterised in that ketones of the formula (IV)

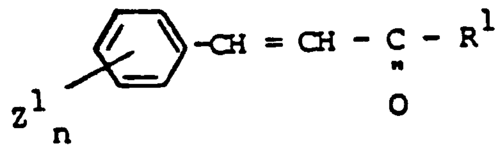 (IV)

in which
$R^1$, $Z^1$ and n have the abovementioned meaning,
either
a) are reacted with dimethyloxosulphonium methylide of the formula (V)

$$\overset{\delta+\ \delta-}{(CH_3)_2SOCH_2} \qquad (V)$$

in the presence of a diluent,
or
b) are reacted with trimethylsulphonium methyl sulphate of the formula (VI)

$$\left[(CH_3)_3S\right]^{(+)} CH_3SO_4^{(-)} \qquad (VI)$$

in the presence of an inert organic solvent and in the presence of a base.

**Revendications**

1. Dérivés du 1-hydroxyéthyl-azole de formule (I)

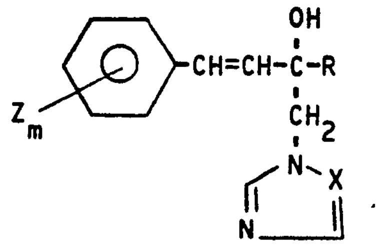

(I)

dans laquelle

R représente un groupe alkyle en $C_1$-$C_4$, un groupe cycloalkyle contenant 3 à 7 atomes de carbone et éventuellement substitué par un groupe alkyle en $C_1$ ou $C_2$, ou un groupe phényle éventuellement substitué par des halogènes, des groupes alkyle en $C_1$-$C_4$ et/ou halogénoalkyle contenant 1 ou 2 atomes de carbone et 1 à 5 atomes d'halogènes,

X représente un atome d'azote ou le groupe CH,

Z représente un halogène, un groupe alkyle en $C_1$-$C_4$, cycloalkyle en $C_5$-$C_7$, alcoxy en $C_1$-$C_4$, alkylthio en $C_1$-$C_4$, halogénoalkyle contenant 1 ou 2 atomes de carbone et 1 à 5 atomes d'halogènes, halogénoalcoxy contenant 1 ou 2 atomes de carbone et 1 à 5 atomes d'halogènes, halogénoalkylthio contenant 1 ou 2 atomes de carbone et 1 à 5 atomes d'halogènes, phényle éventuellement substitué par des halogènes et/ou des groupes alkyle en $C_1$-$C_4$, phénoxy éventuellement substitué par des halogènes et/ou des groupes alkyle en $C_1$-$C_4$, phénylalkyle contenant 1 ou 2 atomes de carbone dans la partie alkyle et éventuellement substitué par des halogènes et/ou des groupes alkyle en $C_1$-$C_4$ ou phénylalcoxy contenant 1 ou 2 atomes de carbone dans la partie alcoxy et éventuellement substitué par des halogènes et/ou des groupes alkyle en $C_1$-$C_4$, et

m est égal à 0, 1, 2 ou 3,

ainsi que leurs sels formés par addition avec des acides et complexes de sels métalliques.

2. Procédé de préparation des dérivés du 1-hydroxyéthyl-azole de formule (I)

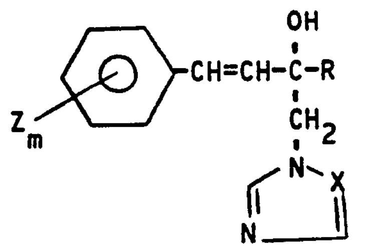

(I)

dans laquelle

R représente un groupe alkyle en $C_1$-$C_4$, cycloalkyle en $C_3$-$C_7$ éventuellement substitué par un groupe alkyle en $C_1$ ou $C_2$ , ou phényle éventuellement substitué par des halogènes, des groupes alkyle en $C_1$-$C_4$ et/ou halogénoalkyle contenant 1 ou 2 atomes de carbone et 1 à 5 atomes d'halogènes,

X représente un atome d'azote ou le groupe CH,

Z représente un halogène, un groupe alkyle en $C_1$-$C_4$, cycloalkyle en $C_5$-$C_7$, alcoxy en $C_1$-$C_4$, alkylthio en $C_1$-$C_4$, halogénoalkyle contenant 1 ou 2 atomes de carbone et 1 à 5 atomes d'halogènes, halogénoalcoxy contenant 1 ou 2 atomes de carbone et 1 à 5 atomes d'halogènes, halogénoalkylthio contenant 1 ou 2 atomes de carbone et 1 à 5 atomes d'halogènes, phényle éventuellement substitué par des halogènes et/ou des groupes alkyle en $C_1$-$C_4$, phénoxy éventuellement substitué par des halogènes et/ou des groupes alkyle en $C_1$-$C_4$, phénylalkyle contenant 1 ou 2 atomes de carbone dans la partie alkyle et éventuellement substitué par des halogènes et/ou des groupes alkyle en $C_1$-$C_4$, ou phénylalcoxy contenant 1 ou 2 atomes de carbone dans la partie alcoxy et éventuellement substitué par des halogènes et/ou des groupes alkyle en $C_1$-$C_4$, et

m est égal à 0, 1, 2 ou 3,

et de leurs sels formés par addition et complexes de sels métalliques,

caractérisé en ce que l'on fait réagir des oxirannes de formule (II)

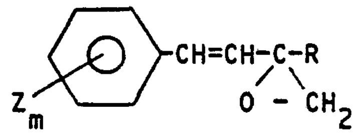

(II)

dans laquelle
R, Z et m ont les significations indiquées ci-dessus, avec des azoles de formule (III)

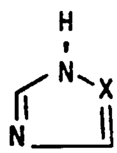

(III)

dans laquelle
X a les significations indiquées ci-dessus,
en présence d'un diluant et, le cas échéant, en présence d'une base, et éventuellement on fixe ensuite par addition un acide ou un sel métalliqée sur les composés de formule (I) ainsi obtenus.

3. Produits régulateurs de la croissance des végétaux et fongicides, caractérisés en ce qu'ils contiennent au moins un dérivé du 1-hydroxyéthyl-azole de formule (I) ou un sel formé par addition avec un acide ou un complexe de sel métallique d'un dérivé du 1-hydroxyéthyl-azole de formule (I), avec des diluants et/ou des substances tensioactives.

4. Utilisation des dérivés du 1-hydroxyéthyl-azole de formule (I) ou de sels formés par addition avec des acides ou des complexes de sels métalliques des dérivés du 1-hydroxyéthyl-azole de formule (I) pour la régulation de la croissance des végétaux ou la lutte contre les mycètes.

5. Procédé de préparation de produits régulateurs de la croissance des végétaux et fongicides, caractérisé en ce que l'on mélange des dérivés du 1-hydroxyéthyl-azole de formule (I) ou des sels formés par addition avec des acides ou des complexes de sels métalliques des dérivés du 1-hydroxyéthyl-azole de formule (I) avec des diluants et/ou des substances tensioactives.

6. Oxirannes de formule (IIa)

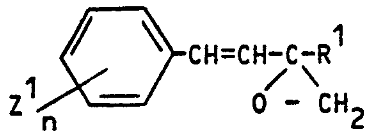

(IIa)

dans laquelle

$R^1$ représente un groupe tert.-butyle, isopropyle ou cyclopropyle éventuellement substitué par un groupe méthyle,

$Z^1$ représente le fluor, le chlore, le brome, un groupe méthyle, tert.butyle, cyclohexyle, méthoxy, méthylthio, trifluorométhyle, trifluorométhoxy, trifluorométhylthio, phényle, phénoxy, benzyle ou benzyloxy, et

n est égal à 0, 1 ou 2.

7. Procédé de préparation des oxirannes de formule (IIa)

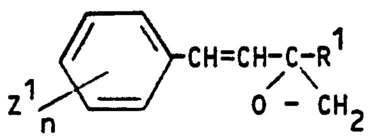

(IIa)

dans laquelle

$R^1$ représente un groupe tert.-butyle, isopropyle ou cyclopropyle éventuellement substitué par un groupe méthyle,

$Z^1$ représente le fluor, le chlore, le brome, un groupe methyle, tert.butyle, cyclohexyle, méthoxy, méthylthio, trifluorométhyle, trifluorométhoxy, trifluorométhylthio, phényle, phénoxy, benzyle ou benzyloxy, et

n est égal à 0, 1 ou 2,

caractérisé en ce que l'on fait réagir des cétones de formule (IV)

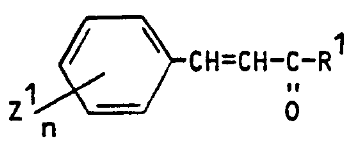

(IV)

dans laquelle

$R^1$, $Z^1$ et n ont les significations indiquées ci-dessus,

a) soit avec le méthylure de diméthyloxosulfonium de formule (V)

$$\overset{\delta+}{}\ \overset{\delta-}{}\quad (CH_3)_2SOCH_2 \qquad (V)$$

en présence d'un diluant,
b) soit avec le méthylsulfate de triméthylsulfonium de formule (VI)

$$\left[(CH_3)_3S^{(+)}\right] CH_3SO_4^{(-)} \qquad (VI)$$

en présence d'un solvant organique inerte et en présence d'une base.